(19) Europäisches Patentamt
European Patent Office
Office européen des brevets

(11) **EP 4 726 724 A1**

(12) **EUROPEAN PATENT APPLICATION**

(43) Date of publication:
**15.04.2026  Bulletin 2026/16**

(21) Application number: **25204061.3**

(22) Date of filing: **23.09.2025**

(51) International Patent Classification (IPC):
***G16C 20/30*** (2019.01)   ***G06N 10/20*** (2022.01)
***G06N 10/60*** (2022.01)   *G16C 10/00* (2019.01)
*G16C 20/70* (2019.01)   *G16C 20/90* (2019.01)

(52) Cooperative Patent Classification (CPC):
**G16C 20/30; G06N 3/045; G06N 3/0464;
G06N 3/08; G06N 3/084; G06N 3/09; G06N 10/00;
G06N 10/20; G06N 10/40; G06N 10/60;
G06N 20/00;** G16C 10/00; G16C 20/70; G16C 20/90

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **26.09.2024  JP 2024167034**

(71) Applicant: **FUJITSU LIMITED
Kawasaki-shi, Kanagawa 211-8588 (JP)**

(72) Inventor: **KAMATA, Yuichi
Kawasaki-shi, 211-8588 (JP)**

(74) Representative: **Haseltine Lake Kempner LLP
Cheapside House
138 Cheapside
London EC2V 6BJ (GB)**

(54) **ESTIMATION PROGRAM, ESTIMATION DEVICE, AND ESTIMATION METHOD FOR ESTIMATING THE PHYSICAL QUANTITY OF A SUBSTANCE**

(57)     Provided is an estimation program causing a computer to execute a process. The process includes receiving proton position information indicating a position of a proton in each of a plurality of atoms included in a first substance as an estimation target and atomic orbital information indicating an atomic orbital of an electron in each of the plurality of atoms, estimating a physical quantity of the estimation target from the proton position information and the atomic orbital information by using a machine learning model, the machine learning model being trained by using training data including information indicating a position of a proton in each of a plurality of atoms included in a second substance and information indicating an atomic orbital of an electron in each of the plurality of atoms included in the second substance, and outputting an estimation result indicating the physical quantity of the estimation target.

FIG. 2

```
          START
            │
   ┌────────▼────────┐
   │ RECEIVE PROTON POSITION INFORMATION AND  │  201
   │    ATOMIC ORBITAL INFORMATION            │
   └────────┬────────┘
            │
   ┌────────▼────────┐
   │ ESTIMATE PHYSICAL QUANTITY OF            │  202
   │     ESTIMATION TARGET                    │
   └────────┬────────┘
            │
   ┌────────▼────────┐
   │ OUTPUT ESTIMATION RESULT                 │  203
   └────────┬────────┘
            │
          END
```

EP 4 726 724 A1

## Description

FIELD

A certain aspect of the present embodiments relates to an estimation program, an estimation device, and an estimation method.

BACKGROUND

Quantum chemical calculation is a technique for analyzing the structure and properties of a compound by theoretical calculation based on quantum mechanics. The quantum chemical calculation is used, for example, to calculate the energy of a molecule in its ground or excited state. As one of techniques for calculating an energy of a ground state using a quantum computer, a variational quantum eigensolver (VQE) is known.

The quantum computer is a computer that realizes calculation by using the phenomenon of quantum mechanics. The quantum computer changes a quantum state of a qubit by using a quantum circuit that is a quantum calculation model describing a quantum algorithm.

In the quantum chemical computation, a neural network architecture using self-attention is known (see, for example, Non-patent literature 1: I. von Glehn et al., "A Self-Attention Ansatz for Ab-initio Quantum Chemistry", arXiv:2211. 13672v2, 2023). A meta-variational quantum eigensolver is also known (see, for example, Non-patent literature 2: A. Cervera-Lierta et al., "Meta-Variational Quantum Eigensolver: Learning Energy Profiles of Parameterized Hamiltonians for Quantum Simulation", PRX QUANTUM 2, 020329, 2021). A quantum self-attention network (QSAN) is also known (see, for example, Non-patent literature 3: J. Shi et al., "QSAN: A Near-term Achievable Quantum Self-Attention Network", arXiv:2207. 07563v4, 2023.).

An explainable transducer transformer is also known (see, for example, Japanese National Publication of International Patent Application No. 2024-500182).

SUMMARY

In the technique of Non-patent literature 1 or Non-patent literature 2, it is difficult to estimate the energy of the ground state of various molecules by a single model.

Such a problem occurs not only in the case of estimating the energy of the ground state of the molecule but also in the case of estimating various physical quantities of various substances.

In one aspect, the present disclosure is directed to estimating a physical quantity according to a type of a substance.

According to an aspect of the present disclosure, there is provided an estimation program causing a computer to execute a process. The process includes: receiving proton position information indicating a position of a proton in each of a plurality of atoms included in a first substance as an estimation target and atomic orbital information indicating an atomic orbital of an electron in each of the plurality of atoms; estimating a physical quantity of the estimation target from the proton position information and the atomic orbital information by using a machine learning model, the machine learning model being trained by using training data including information indicating a position of a proton in each of a plurality of atoms included in a second substance and information indicating an atomic orbital of an electron in each of the plurality of atoms included in the second substance; and outputting an estimation result indicating the physical quantity of the estimation target.

BRIEF DESCRIPTION OF THE DRAWINGS

FIG. 1 is a functional configuration diagram of an estimation device according to an embodiment.
FIG. 2 is a flowchart illustrating a first estimation process.
FIG. 3 is a configuration diagram of a quantum computer system.
FIG. 4 is a diagram illustrating a first estimation model.
FIG. 5 is a diagram illustrating a transformation module included in a first estimation model.
FIG. 6 is a diagram illustrating a feature transformation circuit.
FIG. 7 is a diagram illustrating a state transformation circuit.
FIG. 8 is a diagram illustrating an MPS circuit diagram.
FIG. 9 illustrates an HEA circuit for transforming a first unified feature into a second unified feature.
FIG. 10 is a diagram illustrating an HEA circuit that transforms the second combined feature into a third combined feature.
FIG. 11 is a diagram illustrating hardware configuration of a quantum computer.
FIG. 12 is a diagram illustrating functional configuration of a server.
FIG. 13 is a flowchart illustrating a training process.
FIG. 14 is a flowchart illustrating a second estimation process.
FIG. 15 is a diagram illustrating a second estimation model.
FIG. 16 is a diagram illustrating a transformation module included in the second estimation model.
FIG. 17 is a diagram illustrating hardware configuration of an information processing apparatus.

EFFECT

According to one aspect, it is possible to estimate a physical quantity according to a type of a substance.

## DETAILED DESCRIPTION

Hereinafter, embodiments will be described in detail with reference to the drawings.

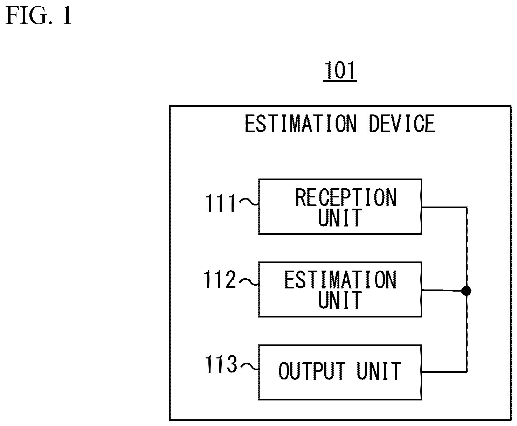

FIG. 1 illustrates an example of a functional configuration of an estimation device according to an embodiment. An estimation device 101 in FIG. 1 includes a reception unit 111, an estimation unit 112, and an output unit 113.

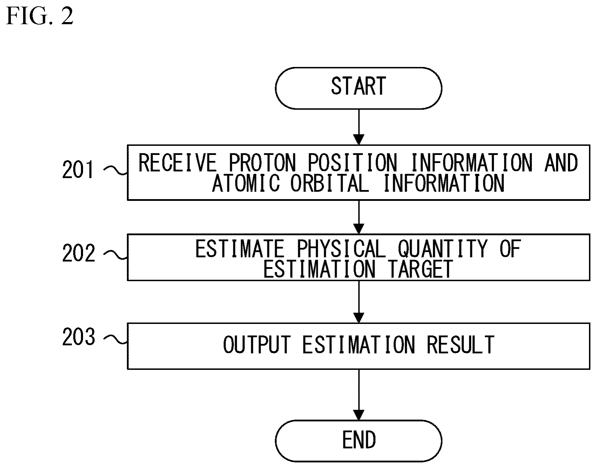

FIG. 2 is a flowchart illustrating an example of a first estimation process performed by the estimation device 101 in FIG. 1. First, the reception unit 111 receives proton position information indicating a position of a proton in each of a plurality of atoms included in an estimation target and atomic orbital information indicating an atomic orbital of an electron in each of the plurality of atoms (step 201).

Next, the estimation unit 112 estimates a physical quantity of the estimation target from the proton position information and the atomic orbital information using a machine learning model trained using training data (step 202). The training data includes information indicating a position of a proton in each of a plurality of atoms included in a substance and information indicating an atomic orbit of an electron in each of the plurality of atoms included in the substance. Then, the output unit 113 outputs an estimation result indicating the physical quantity of the estimation target (step 203).

According to the estimation device 101 of FIG. 1, it is possible to estimate the physical quantity according to the type of the substance.

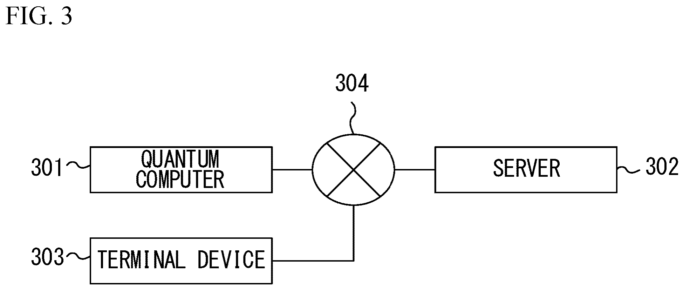

FIG. 3 illustrates an example of a configuration of a quantum computer system including the estimation device 101 illustrated in FIG. 1. The quantum computer system in FIG. 3 includes a quantum computer 301, a server 302, and a terminal device 303. The quantum computer 301, the server 302, and the terminal device 303 are hardware.

The quantum computer system of FIG. 3 is used to estimate the energy of the ground state of the substance in quantum chemical calculation for material development or drug discovery, for example. The energy of the ground state of the substance is an example of the physical quantity of the estimation target.

The server 302 is a classical computer, and the terminal device 303 is an information processing apparatus (computer) of a user. The server 302 corresponds to the estimation device 101 in FIG. 1.

The server 302 communicates with the quantum computer 301 and the terminal device 303 via a communication network 304. The communication network 304 is, for example, a wide area network (WAN) or a local area network (LAN).

The server 302 stores an estimation model to be trained. In the training process, the server 302 generates a trained estimation model by executing machine learning for training the estimation model using the quantum computer 301. The estimation model is an example of a machine learning model.

The server 302 trains the estimation model using the training data. As the training data, for example, proton arrangement information and atomic orbital information of various substances are used. The server 302 calculates the ground state of the substance from the proton arrangement information and the atomic orbital information included in the training data by using the estimation model to be trained. The proton arrangement information is an example of proton position information.

In the estimation process for a substance as the estimation target, the server 302 receives, for example, the proton arrangement information and the atomic orbital information of the substance as the estimation target from the terminal device 303. Then, the server 302 calculates the ground state of the substance from the proton arrangement information and the atomic orbital information of the substance as the estimation target using the trained estimation model.

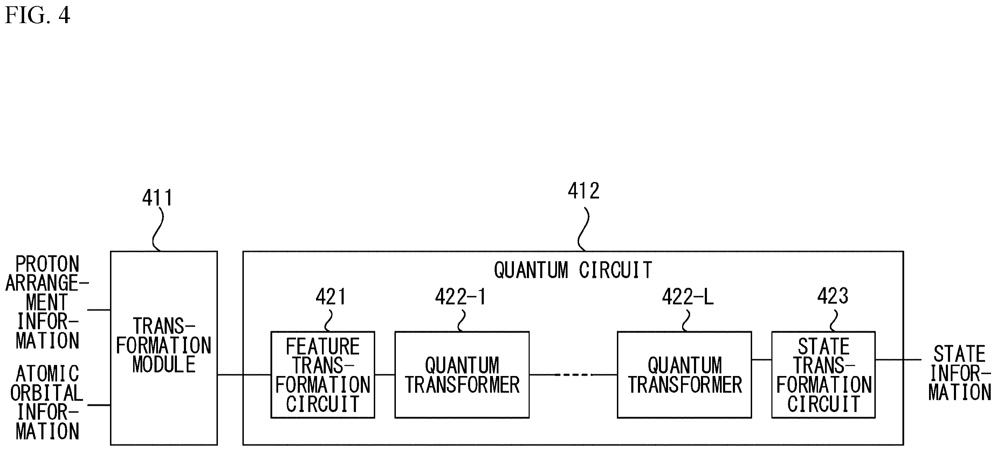

FIG. 4 illustrates an example of the first estimation model. The estimation model of FIG. 4 includes a transformation module 411 and a quantum circuit 412, and calculates the ground state of a substance A from the proton arrangement information and the atomic orbital information of the substance A. The substance A is a substance represented by training data or a substance as the estimation target. The substance A may be a molecule, a compound, or an ion. The ground state of the substance A is an example of a state of the estimation target.

The proton arrangement information indicates the position of each proton included in the atomic nucleus of each of a plurality of atoms included in the substance A. As the proton arrangement information, for example, three dimensional coordinates $(x, y, z)$ of each proton are used. When the substance A includes m protons (m is an integer of 2 or more), the proton arrangement information includes m three dimensional coordinates $(x, y, z)$.

According to such proton arrangement information, a difference in nuclides of the atoms included in the substances represented by the plurality of respective pieces of training data can be uniformly expressed using the number m of protons.

The atomic orbital information indicates an atomic orbital of each electron included in each of a plurality of atoms included in the substance A. The atomic orbitals are represented by wave functions describing the distribution of electrons in the atom and are distinguished using symbols such as 1s, 2s, and 2p.

As the atomic orbital information, for example, an orbital ID that is a number assigned in an ascending order of an energy level of the atomic orbital is used. The orbital ID is a common number that does not depend on the nuclide. When the substance A includes n (n is an integer of 2 or more) electrons, the atomic orbital information includes n orbital IDs. Each orbital ID is represented by, for example, a one hot vector.

As an example, when the substance A is a molecule of lithium hydride LiH, m = n = 4 is satisfied. In this case, the proton arrangement information includes the three dimensional coordinates of each of three protons included in the atomic nucleus of the lithium atom Li and the three dimensional coordinates of one proton included in the atomic nucleus of the hydrogen atom H.

The atomic orbital information includes orbital IDs "1" to "3" of three electrons included in the lithium atom Li and an orbital ID "1" of one electron included in the hydrogen atom H. The orbital ID "1" and the orbital ID "2" represent a 1s orbit, and the orbital ID "3" represents a 2s orbit.

As another example, when the substance A is molecules of beryllium hydride $BeH_2$, m = n = 6 is satisfied. In this case, the proton arrangement information includes the three dimensional coordinates of each of four protons included in the atomic nucleus of the beryllium atom Be and the three dimensional coordinates of one proton included in the atomic nucleus of each of two hydrogen atoms H.

The atomic orbital information includes orbital IDs "1" to "4" of four electrons included in the beryllium atom Be and an orbital ID "1" of one electron included in each of two hydrogen atoms H. The orbital ID "1" and the orbital ID "2" represent the 1s orbit, and the orbital ID "3" and the track ID "4" represent the 2s orbit.

According to such atomic orbital information, the initial position of each electron included in the substance A is associated with the orbital ID corresponding to the energy level of the atomic orbital, and thus the initial position of the electron can be uniformly expressed using the orbital ID.

The transformation module 411 transforms the proton arrangement information and the atomic orbital information into the feature information of the substance A. The feature information of the substance A includes a feature vector representing a feature of each of m × n combinations of protons and electrons included in the substance A.

The quantum circuit 412 represents an operation of generating state information indicating a ground state of the substance A from the feature information of the substance A, and includes a feature transformation circuit 421, quantum transformers 422 - 1 to 422 - L (L is an integer equal to or greater than 1), and a state transformation circuit 423.

The feature transformation circuit 421 represents an operation of transforming the feature information of the substance A into quantum feature information. Each quantum transformer 422 - s (s = 1 to L) includes a self-attention circuit and represents an operation of transforming the quantum feature information.

As the quantum transformer 422 - s, for example, the QSAN of Non-patent literature 3 can be used. The QSAN includes quantum gates of unitary transformations that compute each of Query, Key, and Value, and each quantum gate includes a parameter. The parameter included in the QSAN is an example of a second parameter.

The state transformation circuit 423 represents an operation of transforming the quantum feature information output from the quantum transformer 422 - L into state information indicating the ground state of the n-electron system.

The server 302 generates the feature information of the substance A from the proton arrangement information and the atomic orbital information using the transformation module 411, and causes the quantum computer 301 to execute the operation represented by the quantum circuit 412, thereby acquiring the state information of the substance A from the quantum computer 301. Then, the server 302 calculates the energy of the ground state of the substance A using the state information of the substance A.

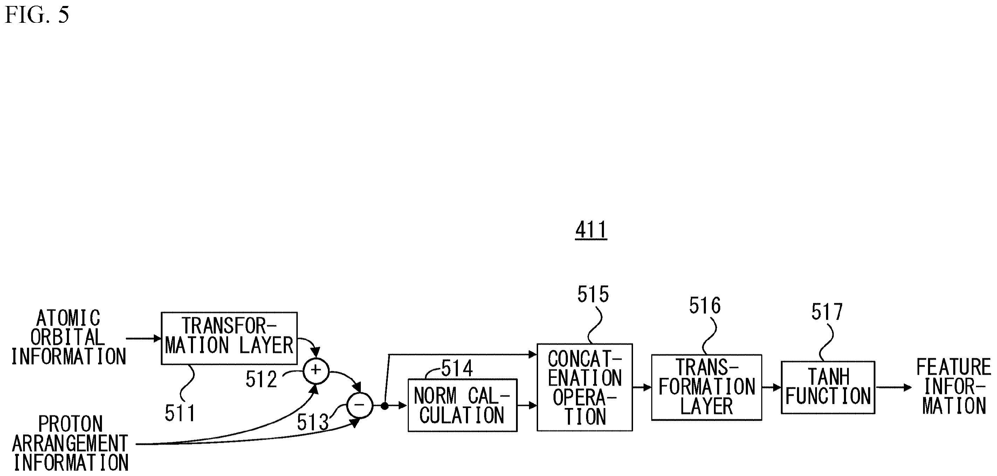

FIG. 5 illustrates an example of the transformation module 411 of FIG. 4. The transformation module 411 of FIG. 5 includes a transformation layer 511, addition 512, subtraction 513, norm calculation 514, concatenation operation 515, a transformation layer 516, and a tanh function 517.

The transformation layer 511 transforms the orbital ID of the j-th (j = 1 to n) electron E (j) included in the atomic orbital information into a three dimensional vector V (j) representing the relative position of the electron with respect to the atomic nucleus. The transformation layer 511 calculates n three dimensional vectors V (j). The transformation layer 511 may be, for example, a neural network including parameters. The transformation layer 511 is an example of a first module, and the parameters included in the transformation layer 511 are an example of a first parameter.

By using the three dimensional vector V (j) representing the relative position of the electron, even if the positions of the protons included in the substances represented by the plurality of training data are different from each other, the position of the electron can be changed according to the difference in the position of the proton. Further, by optimizing the parameters of the transformation layer 511 by training, it is possible to accurately calculate the three dimensional vector V (j) of the n-electron system included in various substances.

The addition 512 adds the three dimensional vector V (j) output from the transformation layer 511 to the three dimensional coordinates of the i-th (i = 1 to m) proton P (i) included in the proton arrangement information. Thus, the three dimensional coordinates of the electron E (j) included in the combination of the proton P (i) and the electron E (j) are obtained.

The subtraction 513 subtracts the three dimensional coordinates of the proton P (i) from the three dimensional coordinates of the electron E (j) obtained by the addition 512. Thus, a three dimensional vector RP (i, j) representing the relative position of the electron E (j) with respect to the proton P (i) is obtained. The subtraction 513 calculates m × n three dimensional vectors RP (i, j). The addition 512 and the subtraction 513 are an example of a second module.

The norm calculation 514 calculates an L2 norm R (i, j) of the three dimensional vector RP (i, j) obtained by the subtraction 513. The norm calculation 514 calculates m × n L2 norms R (i, j). The L2 norm R (i, j) represents a Euclidean distance between the proton P (I) and the electron E (j).

The concatenation operation 515 concatenates the three dimensional vector RP (i, j) and the L2 norm R (i, j) to generate a four dimensional vector V1 (i, j). The concatenation operation 515 generates m × n four dimensional vectors V1 (i, j).

The transformation layer 516 transforms the four dimensional vector V1 (i, j) generated by the concatenation operation 515 into a d-dimensional vector V2 (i, j) (d is an integer of 2 or more). The transformation layer 516 calculates m × n vectors V2 (i, j). The transformation layer 516 may be, for example, a neural network including parameters.

The tanh function 517 is a hyperbolic tangent function, and generate a feature vector FV (i, j) by transforming each of the d elements of the vector V2 (i, j) into a value in the range of - 1 to 1. The tanh function 517 generates m × n feature vectors FV (i, j). The tanh function 517 may also be referred to as an activation function. The norm calculation 514, the concatenation operation 515, the transformation layer 516, and the tanh function 517 are an example of a third module.

The server 302 generates m × n feature vectors FV (I, j) from the atomic orbital information and the proton arrangement information using the transformation module 411. The m × n feature vectors FV (i, j) are used as feature information of the substance A. The server 302 transmits the feature information of the substance A and the information of the quantum circuit 412 to the quantum computer 301.

The quantum computer 301 sets m × n × d qubits to the initial state | 0 >, and then executes the operation represented by the quantum circuit 412 to calculate the measurement value of each qubit. The measurement value of each qubit represents a logical value "0" or a logical value "1".

As an example, assume that material A is molecules of beryllium hydride $BeH_2$. In this case, m = n = 6 is satisfied. The proton P (1) represents a proton included in the first hydrogen atom H, the protons P (2) to P (5) represent four protons included in the atomic nucleus of the beryllium atom Be, and the proton P (6) represents a proton included in the second hydrogen atom H.

The electron E (1) represents an electron included in the first hydrogen atom H, the electrons E (2) to E (5) represent four electrons included in the beryllium atom Be, and the electron E (6) represents an electron included in the second hydrogen atom H.

In this example, the three dimensional coordinates (x, y, z) of the protons P (1) to P (6) included in the proton arrangement information are as follows.

P(1) (0, 0, -2.5)
P(2) (0, 0, 0)
P(3) (0, 0, 0)
P(4) (0, 0, 0)
P(5) (0, 0, 0)
P(6) (0, 0, 2.5)

The orbital IDs of the electrons E (1) to E (6) included in the atomic orbital information are as follows.

E(1) 1
E(2) 1
E(3) 2
E(4) 3
E(5) 4
E(6) 1

The orbital ID "1" and the orbital ID "2" represent the 1s orbit, and the orbital ID "3" and the orbital ID "4" represent the 2s orbit. The one hot vectors representing the orbital ID "1" to the orbital ID "4" are as follows.

1 (1, 0, 0, 0)
2 (0, 1, 0, 0)
3 (0, 0, 1, 0)
4 (0, 0, 0, 1)

The three dimensional vectors V (1) to V (6) output from the transformation layer 511 are as follows.
V (1) = (x (1), y (1), z (1))
V (2) = (x (1), y (1), z (1))
V (3) = (x (2), y (2), z (2))
V (4) = (x (3), y (3), z (3))
V (5) = (x (4), y (4), z (4))
V (6) = (x (1), y (1), z (1))

The three dimensional coordinates of the electrons E (1) to E (6) obtained by the addition 512 are as follows.

E (1) (x (1), y (1), z (1) - 2.5)
E (2) (x (1), y (1), z (1))
E (3) (x (2), y (2), z (2))
E (4) (x (3), y (3), z (3))
E (5) (x (4), y (4), z (4))
E (6) (x (1), y (1), z (1) + 2.5)

The three dimensional vector RP (1, 1) to the three dimensional vector RP (6, 1) of the electron E (1) obtained by the subtraction 513 are as follows.
RP (1, 1) = (x (1), y (1), z (1))
RP (2, 1) = (x (1), y (1), z (1)- 2.5)
RP (3, 1) = (x (1), y (1), z (1) - 2.5)
RP (4, 1) = (x (1), y (1), z (1) - 2.5)
RP (5, 1) = (x (1), y (1), z (1) - 2.5)
RP (6, 1) = (x (1), y (1), z (1) - 5)
The three dimensional vectors RP (1, j) to RP (6, j) of the other electrons E (j) (j = 2 to 6) are similarly obtained. The L2 norm R (1, 1) to the L2 norm R (6, 1) of the electrons E (1) obtained by the norm calculation 514 are as follows.

$$R (1, 1) = (x (1)^2 + y (1)^2 + z (1)^2)^{1/2}$$

$$R (2, 1) = (x (1)^2 + y (1)^2 + (z (1) - 2.5)^2)^{1/2}$$

$$R (3, 1) = (x (1)^2 + y (1)^2 + (z (1) - 2.5)^2)^{1/2}$$

$$R (4, 1) = (x (1)^2 + y (1)^2 + (z (1) - 2.5)^2)^{1/2}$$

$$R (5, 1) = (x (1)^2 + y (1)^2 + (z (1) - 2.5)^2)^{1/2}$$

$$R (6, 1) = (x (1)^2 + y (1)^2 + (z (1) - 5)^2)^{1/2}$$

The L2 norm R (1, j) to the L2 norm R (6, j) of the other electrons E (j) (j = 2 to 6) are similarly obtained. The four dimensional vectors V1 (1, 1) to V1 (6, 1) of the electrons E (1) generated by the concatenation operation 515 are as follows.
V1 (1, 1) = (x (1), y (1), z (1), R (1, 1)
V1 (2, 1) = (x (1), y (1), z (1) - 2.5, R (2, 1))
V1 (3, 1) = (x (1), y (1), z (1) - 2.5, R (3, 1))
V1 (4, 1) = (x (1), y (1), z (1) - 2.5, R (4, 1))
V1 (5, 1) = (x (1), y (1), z (1) - 2.5, R (5, 1))
V1 (6, 1) = (x (1), y (1), z (1) - 5, R (6, 1))

The four dimensional vectors V1 (1, j) to V1 (6, j) of the other electrons E (j) (j = 2 to 6) are similarly obtained.

According to the transformation module 411 of FIG. 5, m × n feature vectors FV (i, j) determined according to the number m of protons and the number n of electrons are generated from the atomic orbital information and the proton arrangement information. By using such a feature vector FV (i, j), it is possible to uniformly express the feature information of various substances input to the quantum circuit 412.

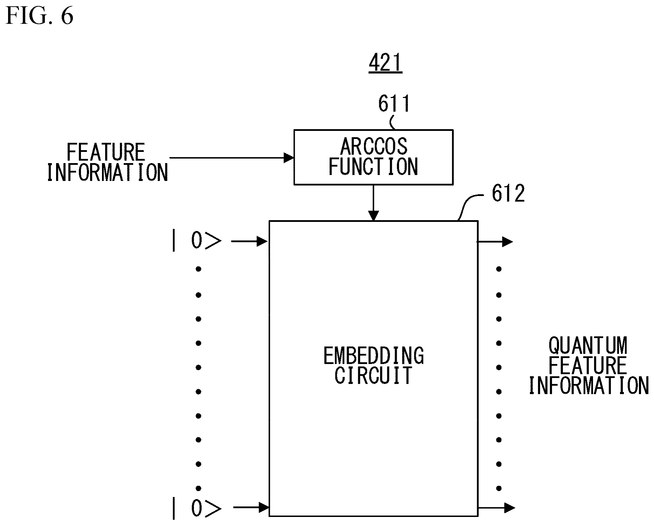

FIG. 6 illustrates an example of the feature transformation circuit 421 of FIG. 4. The feature transformation circuit 421 of FIG. 6 includes an arccos function 611 and an embedding circuit 612.

The arccos function 611 transforms each of d elements of the feature vector FV (i, j) included in the feature information output from the transformation module 411 into an angle, thereby generating m × n × d angles.

The embedding circuit 612 represents rotation operation applied to each of the m × n × d qubits set to the initial state | 0 >. The rotation operation for each qubit is performed using each of the m × n × d angles generated by the arccos function 611. The quantum gate representing the rotation operation may be a Y-rotation gate.

By executing the operation represented by the feature transformation circuit 421, information of m × n feature vectors FV (i, j) is embedded in m × n × d qubits. As a result, quantum feature information including m × n × d quantum features is generated.

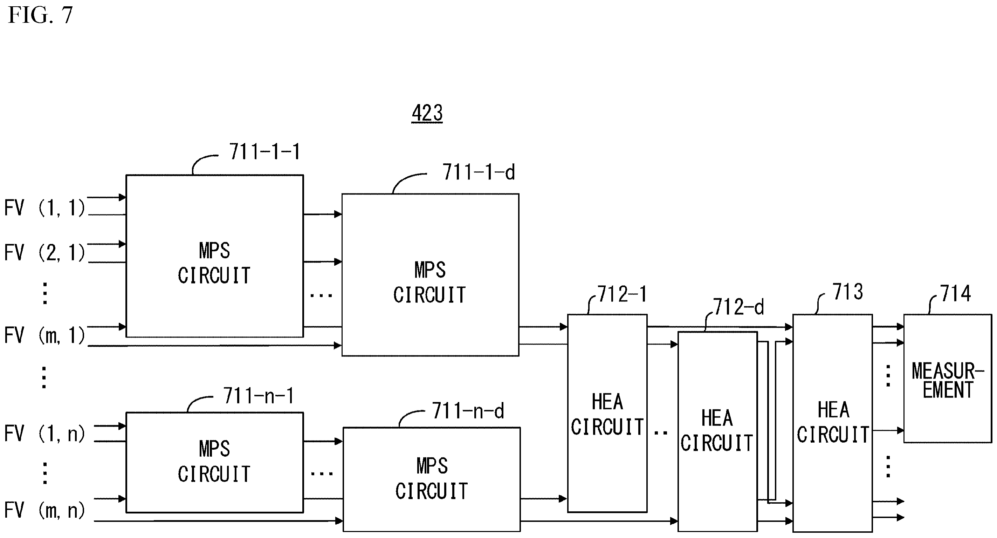

FIG. 7 illustrates an example of the state transformation circuit 423 of FIG. 4. The state transformation circuit 423 in FIG. 7 includes n × d matrix product state (MPS) circuits 711-j-p (j = 1 to n, p = 1 to d), d hardware efficient ansatz (HEA) circuits 712-p (p = 1 to d), and an HEA circuit 713.

Each MPS circuit 711-j-p represents an operation of 2-bond MPS Ansatz that transforms the p-th elements of the feature vectors FV (1, j) to FV (m, j) into first integrated feature of the electron E (j). The first integrated feature of the electron E (j) represents a feature obtained by integrating the p-th elements of the feature vectors FV (1, j) to FV (m, j).

Each HEA circuit 712-p represents an operation of transforming n first integrated features output from the MPS circuits 711-1-p to 711-n-p into n second integrated features. The HEA circuit 713 represents an operation of transforming n × d second integrated features output from the HEA circuit 712-1 to the HEA circuit 712-d into n × d third integrated features.

The quantum computer 301 performs measurement 714 on q qubits, i.e., the first to q-th qubits among the n × d qubits representing the n × d third integrated features, thereby obtaining q measurement values. The quantum computer 301 then transmits the acquired q measurement values to the server 302 as the state information of the substance A.

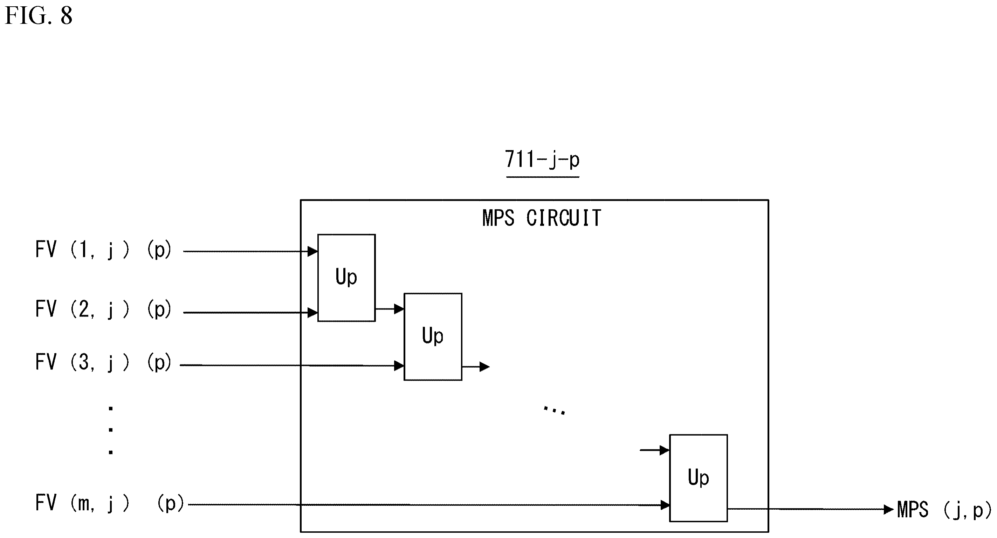

FIG. 8 illustrates an example of the MPS circuit 711-j-p of FIG. 7. The MPS circuit 711-j-p of FIG. 8 includes m-1 Up. Up represents unitary transform and includes 4 × 4 parameters. The parameters included in Up are examples of a second parameter. FV (i, j) (p) (i = 1 to m) represents the p-th element of the feature vector FV (i, j).

The first Up represents unitary transformation for FV (1, j) (p) and FV (2, j) (p). The i-th (i = 2 to m-1) Up represents unitary transform for the transform result by the (i- 1)-th Up and FV (i + 1, j) (p). The transformation result by the (m-1)-th Up is output as the integrated feature MPS (j, p). The integrated feature MPS (j, p) corresponds to the first integrated feature of the electron E (j).

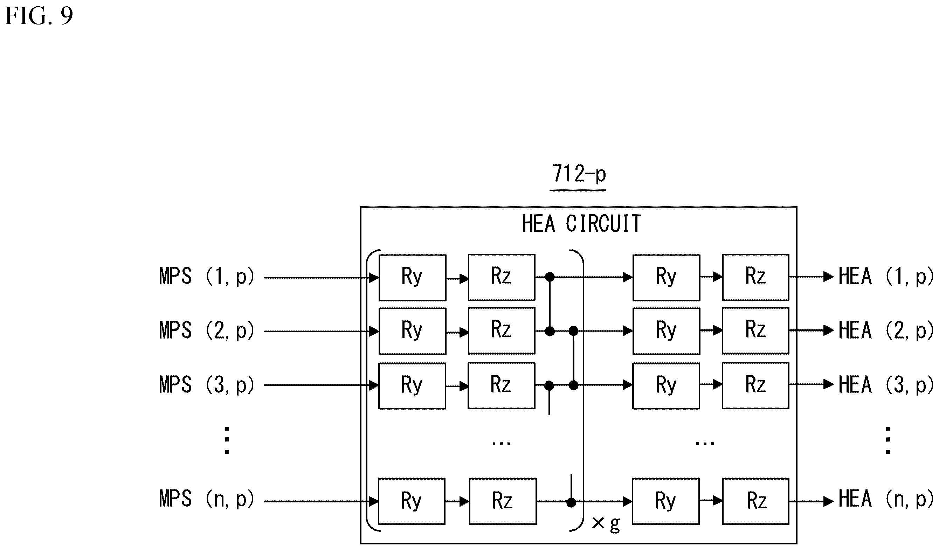

FIG. 9 illustrates an example of the HEA circuit 712-p of FIG. 7. Ry represents the Y rotation gate and Rz represents a Z rotation gate. Ry and Rz include parameters. The parameters included in Ry and Rz are examples of the second parameter. A line segment connecting two adjacent qubits represents a CZ gate. A symbol () × g indicates that circuits in the parentheses are repeated for g stages (g is an integer of 1 or more).

Ry of the first stage circuit transforms the integrated feature MPS (j, p) of the electron E (j), and Rz of the first stage transforms the transformation result by Ry of the first stage. The CZ gate transforms quantum states of two adjacent qubits. The second to g-th circuits also perform the same operation as the first stage circuit.

The transformation result by the g-th stage circuit is transformed by the last Ry, and the transformation result by the last Ry is transformed by the last Rz. The last Rz transform result is output as integrated feature HEA (j, p). The integrated feature HEA (j, p) corresponds to the second integrated feature of the electron E (j).

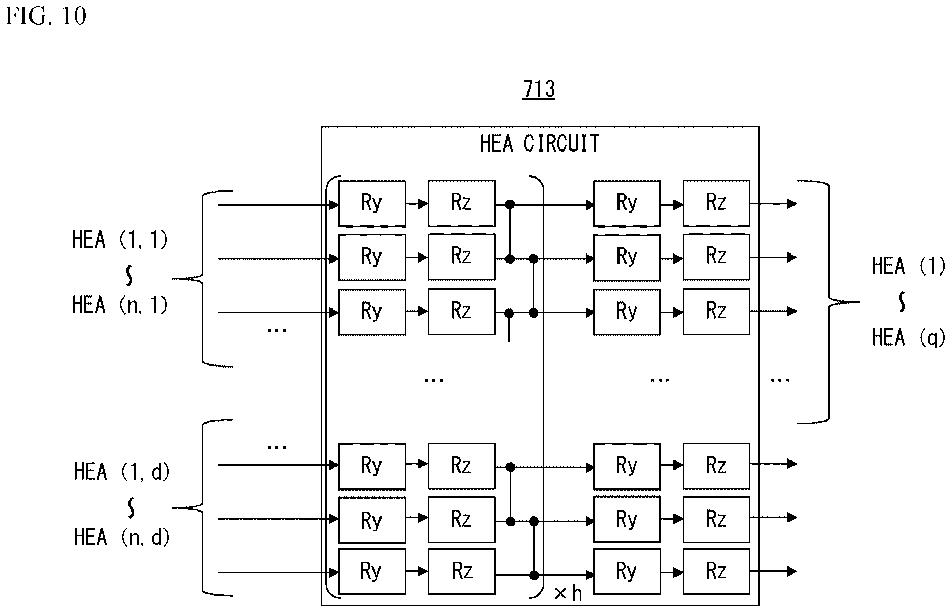

FIG. 10 illustrates an example of the HEA circuit 713 of FIG. 7. Ry represents the Y rotation gate and Rz represents the Z rotation gate. Ry and Rz include parameters. The parameters included in Ry and Rz are examples of the second parameter. A line segment connecting two adjacent qubits represents the CZ gate. A symbol () $\times$ h indicates that circuits in the parentheses are repeated for h stages (h is an integer of 1 or more).

Ry of the first stage circuit transforms the integrated feature HEA (j, p), and Rz of the first stage circuit transforms the transformation result by Ry of the first stage circuit. The CZ gate transforms quantum states of two adjacent qubits. The second stage to h-th stage circuits also represent the same operation as the first stage circuit.

The transformation result by the h-th stage circuit is transformed by the last Ry, and the transformation result by the last Ry is transformed by the last Rz. The transformation result by the last Rz corresponds to the third integrated feature. Among the n $\times$ d last Rz transformation results, the first to q-th q transformation results are output as integrated features HEA (1) to HEA (q).

The quantum computer 301 performs the measurement 714 to obtain the measurement value of each of the integrated features HEA (1) to HEA (q).

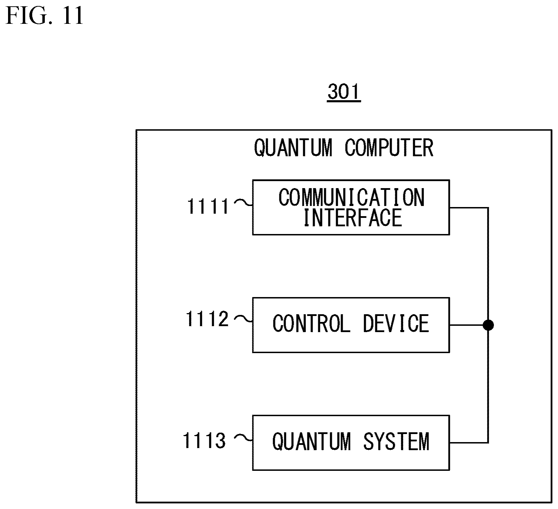

FIG. 11 illustrates an example of the hardware configuration of the quantum computer 301 in FIG. 3. The quantum computer 301 of FIG. 11 includes a communication interface 1111, a control device 1112, and a quantum system 1113. These components are hardware.

The communication interface 1111 is a communication circuit that is connected to the communication network 304 and performs data transformation associated with communication. The quantum system 1113 includes a quantum device that implements a plurality of qubits.

The communication interface 1111 receives the feature information of the substance A and the information of the quantum circuit 412 from the server 302, and outputs the received information to the control device 1112. The control device 1112 generates a control signal using the feature information of the substance A and the information of the quantum circuit 412, and outputs the control signal to the quantum system 1113.

The quantum system 1113 calculates a measurement value of each qubit by operating the qubit according to the control signal. Then, the control device 1112 transmits the measurement value of each qubit obtained by the quantum system 1113 to the server 302 via the communication interface 1111.

The quantum computer 301 executes M measurements 714 by repeating the operation represented by the quantum circuit 412 M times (M is an integer of 1 or more), for example. The quantum computer 301 then transmits the state information obtained in each of the M measurements 714 to the server 302.

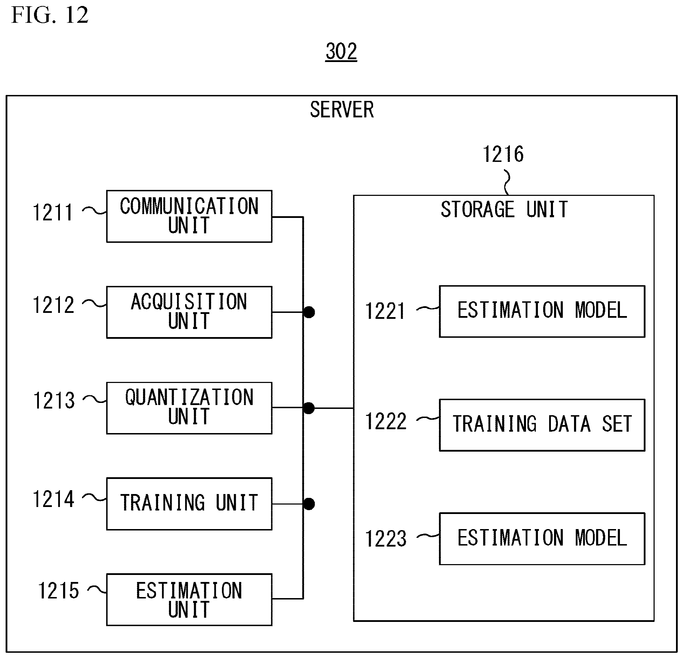

FIG. 12 illustrates an example of the functional configuration of the server 302 in FIG. 3. The server 302 in FIG. 12 includes a communication unit 1211, an acquisition unit 1212, a quantization unit 1213, a training unit 1214, an estimation unit 1215, and a storage unit 1216. The communication unit 1211, the acquisition unit 1212, and the estimation unit 1215 correspond to the output unit 113, the reception unit 111, and the estimation unit 112 in FIG. 1, respectively.

The communication unit 1211 communicates with the quantum computer 301 and the terminal device 303 via the communication network 304. The communication unit 1211 can also communicate with a database server (not illustrated) or the like via the communication network 304. The storage unit 1216 stores an estimation model 1221 to be trained.

The acquisition unit 1212 acquires a training data set 1222 including a plurality of training data from a database server or the like via the communication unit 1211, and stores the training data set in the storage unit 1216. The acquisition unit 1212 may acquire the training data set 1222 input from the operator via a user interface or a portable recording medium.

In the training process, the quantization unit 1213 generates a Hamiltonian HM of an n-electron system of a substance represented by each training data included in the training data set 1222, and maps a ground state of the n-electron system to q qubits.

The training unit 1214 trains an estimation model 1223 using the training data set 1222 while communicating with the quantum computer 301 via the communication unit 1211, thereby generating the trained estimation model 1221 and storing the generated estimation model in the storage unit 1216.

The training unit 1214 generates feature information of a substance from the proton arrangement information and the atomic orbital information included in each training data using the transformation module 411 included in the estimation model 1221. The training unit 1214 transmits the feature information of the substance and the information of the quantum circuit 412 included in the estimation model 1221 to the quantum computer 301 via the communication unit 1211, and receives M pieces of state information of the substance from the quantum computer 301.

A general Hamiltonian HM is expressed by a linear combination of products of a plurality of Pauli operators. In this case, the training unit 1214 can calculate an expected value of the Hamiltonian HM using q logical values included in each of the received M pieces of state information. The expected value of the calculated Hamiltonian HM represents the energy of the ground state of the substance.

The training unit 1214 updates the parameters of the transformation module 411 and the quantum circuit 412 so that the expected value of the Hamiltonian HM decreases using a backpropagation method. The parameters of the transformation module 411 are parameters included in the transformation layer 511 and the transformation layer 516. The parameters of the quantum circuit 412 are parameters included in the quantum transformer 422-s, the MPS circuit 711-j-p, the HEA circuit 712-p, and the HEA circuit 713.

The training unit 1214 can update each parameter by calculating a gradient using, for example, a parameter shift method. The training unit 1214 determines the value of each parameter by, for example, repeating the update of the parameter over N epochs (N is an integer of 1 or more). Then, the training unit 1214 sets the determined parameter values in the transformation module 411 and the quantum circuit 412, thereby generating the trained estimation model 1223.

By optimizing the parameters of the transformation layer 511 by training, it is possible to calculate the feature information of various substances with high accuracy. Further, by optimizing the parameters of the quantum circuit 412 by training, it is possible to calculate the state information of various substances with high accuracy.

In the estimation process, the acquisition unit 1212 acquires the proton arrangement information and the atomic orbital information of the substance of the estimation target from the terminal device 303 via the communication unit 1211, and receives the acquired proton arrangement information and the acquired atomic orbital information. The acquisition unit 1212 may acquire the proton arrangement information and the atomic orbital information input from the operator via the user interface or the portable recording medium.

The quantization unit 1213 generates the Hamiltonian HM of the n-electron system of the substance of the estimation target, and maps the ground state of the n-electron system to q qubits.

The estimation unit 1215 estimates the energy of the ground state of the substance of the estimation target using the estimation model 1223 while communicating with the quantum computer 301 via the communication unit 1211, and generates an estimation result indicating the estimated energy. Then, the estimation unit 1215 transmits the estimation result to the terminal device 303 via the communication unit 1211. The terminal device 303 displays the received estimation result on a screen.

The estimation unit 1215 generates the feature information of the substance of the estimation target from the proton arrangement information and the atomic orbital information of the substance by using the transformation module 411 included in the estimation model 1223.

The estimation unit 1215 transmits the feature information of the substance of the estimation target and the information of the quantum circuit 412 included in the estimation model 1223 to the quantum computer 301 via the communication unit 1211, and receives M pieces of state information of the substance of the estimation target from the quantum computer 301.

Then, the estimation unit 1215 calculates the expected value of the Hamiltonian HM using q logical values included in each of the received M pieces of state information. The expected value of the calculated Hamiltonian HM represents the energy of the ground state of the substance of the estimation target.

By causing the quantum computer 301 to execute the operation represented by the quantum circuit 412 among the transformation module 411 and the quantum circuit 412 included in the estimation model 1223, the state information of the substance of the estimation target can be calculated at high speed.

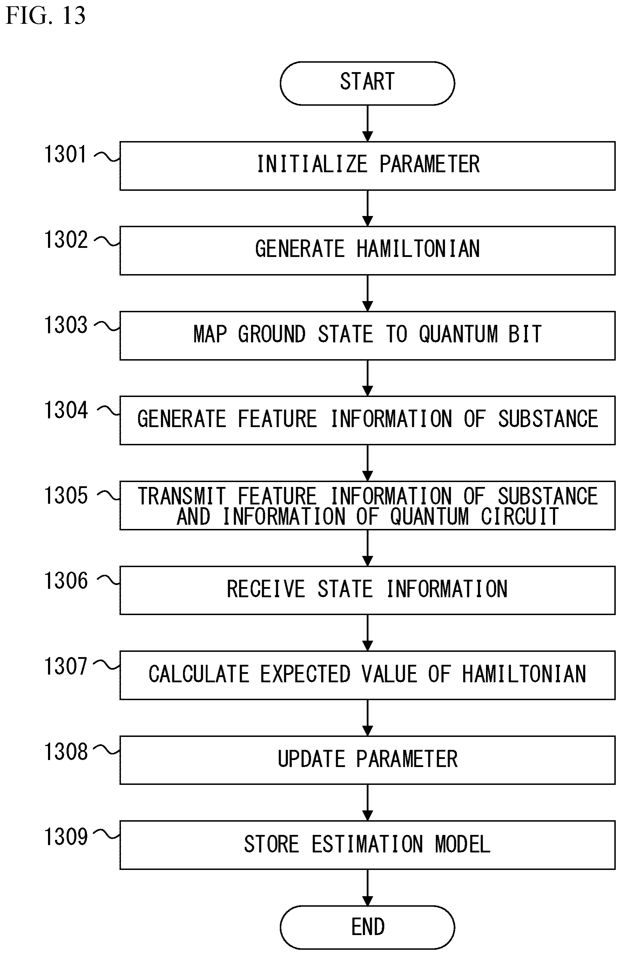

FIG. 13 is a flowchart illustrating an example of a training process performed by the server 302 in FIG. 12. First, the training unit 1214 initializes the parameters of the transformation module 411 and the quantum circuit 412 included in the estimation model 1221 (step 1301).

Next, the server 302 repeats the process of the training loop of step 1302 to step 1308 N times. N represents the number of epochs.

In the process of the training loop, the quantization unit 1213 generates the Hamiltonian HM of the n-electron system of the substance represented by each training data included in the training data set 1222 (step 1302). Then, the quantization unit 1213 maps the ground state of the n-electron system to q qubits (step 1303).

Next, the training unit 1214 generates the feature information of the substance from the proton arrangement information and the atomic orbital information included in each training data by using the transformation module 411 included in the estimation model 1221 (step 1304). Then, the training unit 1214 transmits the feature information of the substance and the information of the quantum circuit 412 included in the estimation model 1221 to the quantum computer 301 via the communication unit 1211 (step 1305).

The quantum computer 301 generates M pieces of state information of the substance by repeating the operation represented by the quantum circuit 412 M times using the feature information of the substance. The quantum computer 301 then transmits the generated M pieces of state information to the server 302.

The training unit 1214 receives M pieces of state information from the quantum computer 301 via the communication unit 1211 (step 1306). Then, the training unit 1214 calculates the expected value of the Hamiltonian HM using q logical values included in each of the received M pieces of state information (step 1307).

Next, the training unit 1214 updates the parameters of the transformation module 411 and the quantum circuit 412 so that the expected value of the Hamiltonian HM decreases using the backpropagation method (step 1308).

After the process of the training loop is executed N times, the training unit 1214 stores the estimation model 1221 including the updated parameters in the storage unit 1216 as the estimation model 1223 (step 1309).

In the process of each training loop, the training unit 1214 may use all the training data included in the training data set 1222 or may use some of the training data. In the process of each training loop, the training unit 1214 may randomly select the training data.

As an example, when the training data set 1222 includes training data representing molecules of hydrogen $H_2$, hydrogenated beryllium $BeH_2$, and two hydrogen molecules arranged in a rectangle with an intermolecular distance $H_4$, the training unit 1214 randomly selects one molecule from among the molecules in the process of each training loop. The training unit 1214 then sets a bond length between atoms of the selected molecule to a random value, thereby determining the position of each proton included in the molecule. As a result, different training data is generated for each training loop.

The estimation model 1221 is trained using the training data of molecules of hydrogen $H_2$, hydrogenated beryllium $BeH_2$, and two hydrogen molecules arranged in a rectangle with an intermolecular distance $H_4$ having various bond lengths, and thus the estimation model 1223 that estimates the energy of the ground state of various substances can be generated.

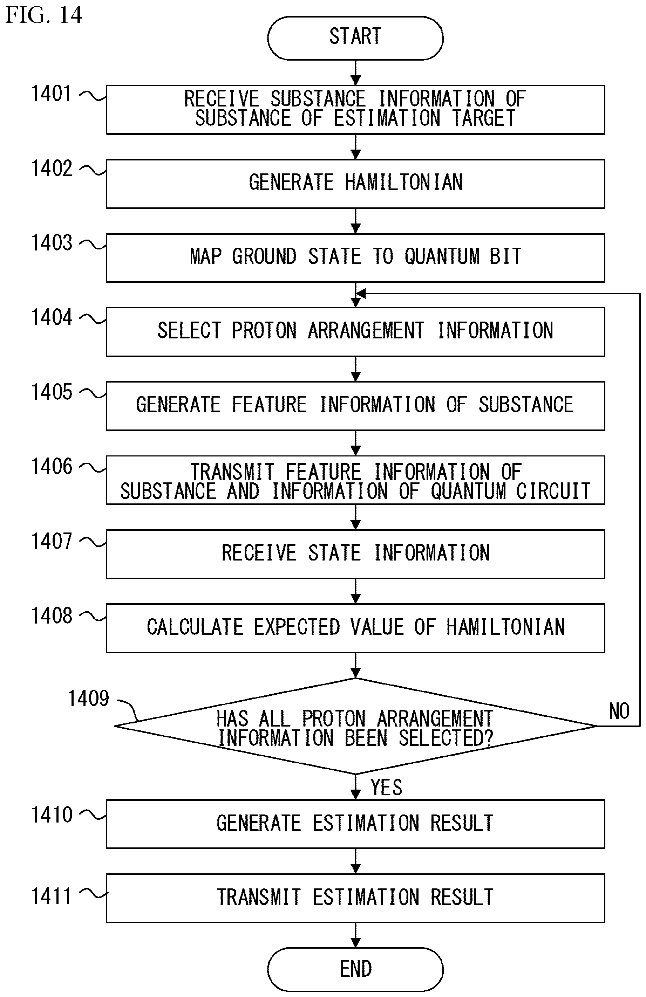

FIG. 14 is a flowchart illustrating an example of the second estimation process performed by the server 302 of FIG. 12. First, the acquisition unit 1212 receives the substance information of the substance of the estimation target from the terminal device 303 via the communication unit 1211 (step 1401).

The substance information includes a plurality of pieces of proton arrangement information and the atomic orbital information. Each piece of proton arrangement information represents the arrangement of a plurality of protons included in the substance of the estimation target, and the plurality of pieces of proton arrangement information represent respective different arrangements. The arrangements of the plurality of protons correspond to the arrangements of a plurality of atoms included in the substance of the estimation target.

Next, the quantization unit 1213 generates the Hamiltonian HM of the n-electron system of the substance of the estimation target (step 1402). Then, the quantization unit 1213 maps the ground state of the n-electron system to q qubits (step 1403).

Next, the estimation unit 1215 selects one piece of proton arrangement information from among the plurality of pieces of received proton arrangement information (step 1404). Then, the estimation unit 1215 generates the feature information of the substance of the estimation target from the selected proton arrangement information and the atomic orbital information using the transformation module 411 included in the estimation model 1223 (step 1405).

Next, the estimation unit 1215 transmits the feature information of the substance of the estimation target and the information of the quantum circuit 412 included in the estimation model 1223 to the quantum computer 301 via the communication unit 1211 (step 1406).

The quantum computer 301 generates M pieces of state information of the substance by repeating the operation represented by the quantum circuit 412 M times using the feature information of the substance. The quantum computer 301 then transmits the generated M pieces of state information to the server 302.

The estimation unit 1215 receives M pieces of state information from the quantum computer 301 via the communication unit 1211 (step 1407). Then, the estimation unit 1215 calculates the expected value of the Hamiltonian HM using q logical values included in each of the received M pieces of state information (step 1408).

Next, the estimation unit 1215 checks whether all the proton arrangement information has been selected (step 1409). When there is any unselected proton placement information (NO in step 1409), the estimation unit 1215 repeats the process in step 1404 and the subsequent steps for the next proton placement information.

When all the proton arrangement information has been selected (step 1409, YES), the estimation unit 1215 generates an estimation result including the expected value of the Hamiltonian HM obtained using each of the plurality of proton arrangement information (step 1410). Then, the estimation unit 1215 transmits the estimation result to the terminal device 303 via the communication unit 1211 (step 1411).

The terminal device 303 generates a space for drawing an energy surface of the ground state of the substance of the estimation target, using a variable for distinguishing the arrangement of the plurality of atoms represented by each piece of proton arrangement information. As the variable for distinguishing the arrangement of the plurality of atoms, a bond length between two atoms, a bond angle formed by three atoms, or the like is used.

The terminal device 303 plots each expected value included in the estimation result at a position corresponding to the value of each variable in the generated space, thereby generating an energy surface of the ground state and displaying the generated energy surface on the screen.

According to the quantum computer system of FIG. 3, since various substances such as molecules, compounds, and ions are expressed using common information such as the number m of protons, the number n of electrons, and the orbital ID of each electron, the versatility of the estimation model 1223 is increased. Therefore, by calculating the energy of the ground state using the estimation model 1223, the energy of the ground state can be efficiently estimated according to the type of the substance.

As an example, it is assumed that the estimation model 1223 is generated using the training data of molecules of hydrogen $H_2$, hydrogenated beryllium $BeH_2$, and two hydrogen molecules arranged in a rectangle with an intermolecular distance $H_4$ having various bond lengths. In this case, the energy of the ground state of the molecule of lithium hydride LiH having a molecular structure different from that of the training data can be estimated using the generated estimation model 1223.

A hydrogen atom H contains one proton, a beryllium atom Be contains four protons and a lithium atom Li contains three protons. The number of protons of the lithium atom Li is larger than the number of protons of the hydrogen atom H and smaller than the number of protons of the beryllium atom Be.

In this case, by using the generated estimation model 1223, an effect of interpolating the state of the Li-H bond using the state of the H-H bond and the state of the Be-H bond is obtained. Therefore, even for a molecule of lithium hydride LiH having a molecular structure different from that of the training data, an effective estimated value of the energy of the ground state can be calculated.

The user can analyze a reaction rate in the chemical reaction, a change in the molecular structure, and the like from the shape of the energy curved surface displayed on the screen. Therefore, by using the quantum computer system of FIG. 3, the efficiency of simulation in the field of material development or drug discovery is improved.

The estimation model 1223 can also be used to estimate other physical quantities other than the energy of the ground state of the substance. The other physical quantity may be the position or momentum of each electron in the ground state, or may be the energy in the excited state.

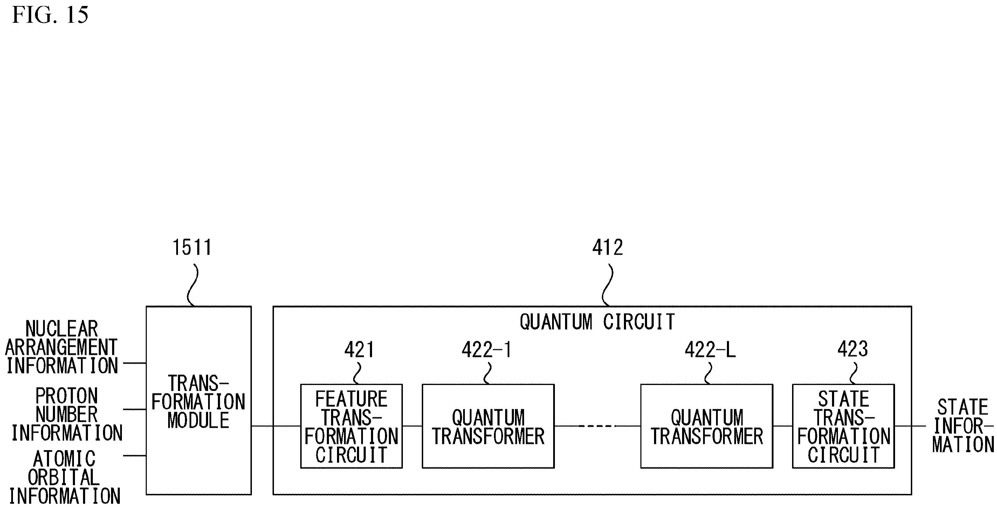

FIG. 15 illustrates an example of a second estimation model. The estimation model in FIG. 15 has a structure in which the transformation module 411 in the estimation model in FIG. 4 is replaced with a transformation module 1511.

Atomic nucleus arrangement information indicates the position of the atomic nucleus of each of a plurality of atoms included in the substance A. As the atomic nucleus arrangement information, for example, three dimensional coordinates (x, y, z) of each atomic nucleus are used. When the substance A includes k (k is an integer of 2 or more) atoms, the atomic nucleus arrangement information includes k three dimensional coordinates (x, y, z). The atomic nucleus arrangement information is an example of the proton position information.

Proton number information indicates the number of protons of each of a plurality of atoms included in the substance A. According to such atomic nucleus arrangement information and proton number information, a difference in nuclides of the atoms included in the substance represented by each of the plurality of training data can be uniformly expressed using the number k of atoms and the number of protons included in each atom.

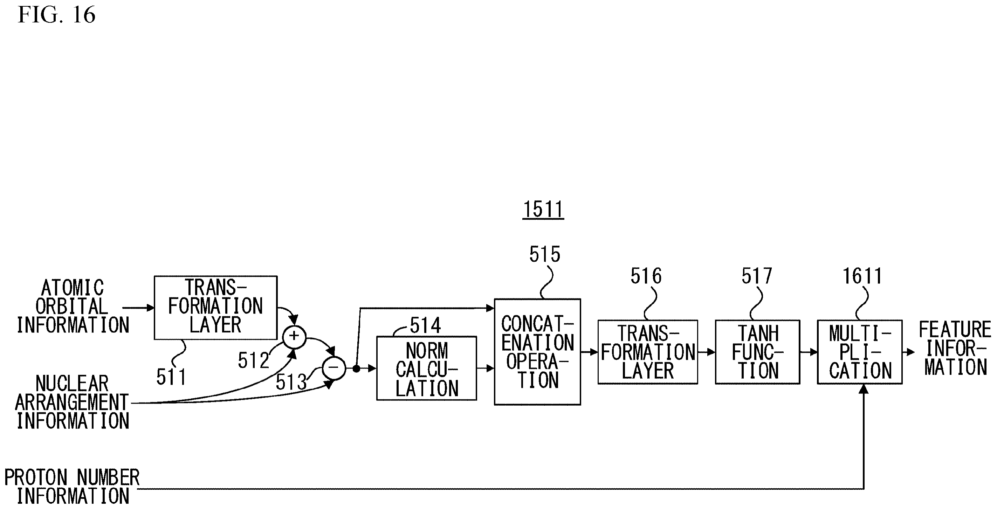

FIG. 16 illustrates an example of the transformation module 1511 of FIG. 15.

The transformation module 1511 of FIG. 16 has a structure in which multiplication 1611 is added to the transformation module 411 of FIG. 5. The proton number information includes the number m (i) of protons included in the atomic nucleus AN (i) (i = 1 to k) of each atom.

The transformation layer 511 transforms the orbital ID of the j-th (j = 1 to n) electron E (j) included in the atomic orbital information into the three dimensional vector V (j) representing the relative position of the electron with respect to the atomic nucleus.

The addition 512 adds the three dimensional vector V (j) output from the transformation layer 511 to the three dimensional coordinates of the i-th atomic nucleus AN (i) included in the atomic nucleus arrangement information. Thus, the three dimensional coordinates of the electron E (j) included in the combination of the atomic nucleus AN (i) and the electron E (j) are obtained.

The subtraction 513 subtracts the three dimensional coordinates of the atomic nucleus AN (i) from the three dimensional coordinates of the electron E (j) obtained by the addition 512. Thus, the three dimensional vector RP (i, j) representing the relative position of the electron E (j) with respect to the atomic nucleus AN (i) is obtained. The subtraction 513 calculates k × n three dimensional vectors RP (i, j).

The norm calculation 514 calculates the L2 norm R (i, j) of the three dimensional vector RP (i, j) obtained by the subtraction 513. The norm calculation 514 calculates k × n L2 norms R (i, j). The L2 norm R (i, j) represents the Euclidean distance between the atomic nucleus AN (i) and the electron E (j).

The concatenation operation 515 concatenates the three dimensional vector RP (i, j) and the L2 norm R (i, j) to generate the four dimensional vector V1 (i, j). The concatenation operation 515 generates k × n four dimensional vectors V1 (i, j).

The transformation layer 516 transforms the four dimensional vector V1 (i, j) generated by the concatenation operation 515 into a d-dimensional vector V2 (i, j). The transformation layer 516 calculates k × n vectors V2 (i, j).

The tanh function 517 generates the feature vector FV (i, j) by transforming each of the d elements of the vector V2 (i, j) to a value in the range of - 1 to 1. The tanh function 517 generates k × n feature vectors FV (i, j).

The multiplication 1611 multiplies the value of each element of the feature vector FV (i, j) by the number m (i) of protons of the atomic nucleus AN (i) included in the proton number information to uniformly amplify the value of each element, thereby generating an amplified feature vector AFV (i, j). The norm calculation 514, the concatenation operation 515, the transformation layer 516, the tanh function 517, and the multiplication 1611 are an example of the third module.

In the training process or the estimation process using the estimation model of FIG. 15, the atomic nucleus arrangement information and the proton number information are used instead of the proton arrangement information. In this case, the server 302 generates $k \times n$ feature vectors AFV (i, j) from the atomic orbital information, the atomic nucleus arrangement information, and the proton number information using the transformation module 1511. The $k \times n$ feature vectors AFV (i, j) are used as the feature information of the substance A. The server 302 transmits the feature information of the substance A and the information of the quantum circuit 412 to the quantum computer 301.

The structure of the quantum circuit 412 is similar to that of the estimation model of FIG. 4. However, the arccos function 611 in FIG. 6 generates $k \times n \times d$ angles by transforming d elements of the feature vector AFV (i, j) into angles instead of the feature vector FV (i, j). The embedding circuit 612 represents the rotation operation applied to each of the $k \times n \times d$ qubits set to the initial state | 0 >.

In this case, each MPS circuit 711-j-p in FIG. 7 represents the operation of the 2-bond MPS Ansatz to transform the p-th elements of the feature vectors AFV (1, j) to AFV (k, j) into the first integrated feature of the electron E (j).

The quantum computer 301 generates M pieces of state information of the substance A by repeating the operation represented by the quantum circuit 412 M times using the feature information of the substance A. The quantum computer 301 then transmits the generated M pieces of state information to the server 302.

According to the transformation module 1511 of FIG. 16, $k \times n$ feature vectors AFV (i, j) determined according to the number k of atomic nuclei and the number n of electrons are generated from the atomic orbital information, the atomic nucleus arrangement information, and the proton number information. The element of each feature vector AFV (i, j) have a value corresponding to the number m (i) of protons of the atomic nucleus AN (i). By using such a feature vector AFV (i, j), it is possible to uniformly express the feature information of various substances input to the quantum circuit 412.

By using $k \times n$ feature vectors AFV (i, j) instead of $m \times n$ feature vectors FV (i, j), the number of qubits used for the operation represented by the quantum circuit 412 is reduced from $m \times n \times d$ to $k \times n \times d$. Therefore, by using the estimation model in FIG. 15, the calculation resources of the quantum computer 301 can be reduced as compared with the case of using the estimation model in FIG. 4.

The server 302 does not necessarily need to estimate the physical quantity of the substance by using the quantum computer 301, and the estimation model does not necessarily need to include the quantum circuit 412. When the quantum computer 301 is not used, the quantum circuit 412 included in the estimation model is replaced with another machine learning model. The another machine learning model may be a neural network or a random forest.

The configuration of the estimation device 101 in FIG. 1 is merely an example, and some of the components may be omitted or changed according to the use or conditions of the estimation device 101.

The configuration of the quantum computer system in FIG. 3 is merely an example, and some of the components may be omitted or changed according to the use or conditions of the quantum computer system. For example, when the estimation model does not include the quantum circuit 412, the quantum computer 301 can be omitted.

The configuration of the quantum computer 301 in FIG. 11 is merely an example, and some of the components may be omitted or changed according to the use or conditions of the quantum computer system.

The configuration of the server 302 in FIG. 12 is merely an example, and some of the components may be omitted or changed according to the use or conditions of the quantum computer system. For example, when the training process is performed by an external device, the training unit 1214 can be omitted.

The flowcharts of FIGs. 2, 13, and 14 are merely examples, and some of the processes may be omitted or changed according to the configuration or conditions of the estimation device 101 or the quantum computer system. For example, when the training process is performed by an external device, the server 302 can omit the training process of FIG. 13.

The structures of the estimation models illustrated in FIGs. 4 to 10, 15, and 16 are merely examples, and the structures of the estimation models change depending on the use or conditions of the quantum computer system.

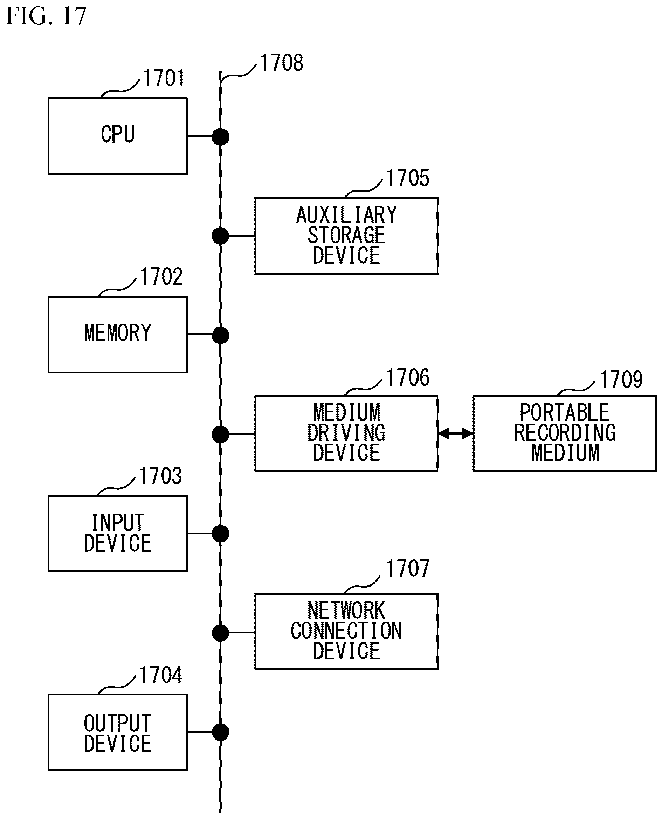

FIG. 17 illustrates an example of the hardware configuration of an information processing apparatus to be used as the estimation device 101 in FIG. 1 and the server 302 in FIG. 12. The information processing apparatus illustrated in FIG. 17 includes a central processing unit (CPU) 1701, a memory 1702, an input device 1703, an output device 1704, an auxiliary storage device 1705, a medium driving device 1706, and a network connection device 1707. These components are hardware and are connected to each other via a bus 1708.

The memory 1702 is, for example, a semiconductor memory such as a read only memory (ROM) or a random access memory (RAM), and stores a program and data used for processes. The memory 1702 may operate as the storage unit 1216 in FIG. 12.

The CPU 1701 (processor) operates as the reception unit 111 and the estimation unit 112 in FIG. 1 by executing a program using the memory 1702, for example. The CPU 1701 also operates as the acquisition unit 1212, the quantization unit 1213, the training unit 1214, and the estimation unit 1215 in FIG. 12 by executing a program using the memory 1702.

The input device 1703 is, for example, a keyboard, a pointing device, or the like, and is used for inputting an instruction or information from an operator. The output device 1704 is, for example, a display device, a printer, or the like, and is used for outputting an inquiry or an instruction to an operator and a processing result. The processing result may be an estimation result.

The auxiliary storage device 1705 is, for example, a magnetic disk device, an optical disk device, a magneto-optical disk device, a tape device, or the like. The auxiliary storage device 1705 may be a hard disk drive or a solid state drive (SSD). The information processing apparatus can store a program and data in the auxiliary storage device 1705, and can load them into the memory 1702 to use them. The auxiliary storage device 1705 may operate as the storage unit 1216 in FIG. 12.

The medium driving device 1706 drives a portable recording medium 1709 and accesses the recorded content. The portable recording medium 1709 is a memory device, a flexible disk, an optical disk, a magneto-optical disk, or the like. The portable recording medium 1709 may be a compact disk read only memory (CD-ROM), a digital versatile disk (DVD), a universal serial bus (USB) memory, or the like. The operator can store the program and the data in the portable recording medium 1709 and load them into the memory 1702 to use them.

As described above, a computer-readable recording medium that stores a program and data used for processes is a physical non-transitory recording medium such as the memory 1702, the auxiliary storage device 1705, or the portable recording medium 1709.

The network connection device 1707 is a communication circuit that is connected to the communication network 304 and performs data transformation associated with communication. The network connection device 1707 may operate as the communication unit 1211 in FIG. 12. The information processing apparatus can receive a program and data from an external apparatus via the network connection device 1707, and load them into the memory 1702 to use them.

Note that the information processing apparatus does not need to include all the components in FIG. 17, and some of the components may be omitted depending on the use or conditions of the information processing apparatus. For example, when an interface with the operator is not necessary, the input device 1703 and the output device 1704 may be omitted. When the portable recording medium 1709 is not used, the medium driving device 1706 may be omitted.

As the terminal device 303 in FIG. 3, an information processing apparatus having a configuration similar to that in FIG. 17 can be used.

Although the embodiment of the present invention is described in detail, the present invention is not limited to the specifically described embodiments and variations but other embodiments and variations may be made without departing from the scope of the claimed invention.

## Claims

1. An estimation program causing a computer to execute a process, the process comprising:

   receiving proton position information indicating a position of a proton in each of a plurality of atoms included in a first substance as an estimation target and atomic orbital information indicating an atomic orbital of an electron in each of the plurality of atoms;
   estimating a physical quantity of the estimation target from the proton position information and the atomic orbital information by using a machine learning model, the machine learning model being trained by using training data including information indicating a position of a proton in each of a plurality of atoms included in a substance and information indicating an atomic orbital of an electron in each of the plurality of atoms included in the second substance; and
   outputting an estimation result indicating the physical quantity of the estimation target.

2. The estimation program according to claim 1,

   wherein the machine learning model includes:

      a transformation module (411, 1511) that transforms the proton position information and the atomic orbital information into feature information of the estimation target; and
      a quantum circuit (412) that generates state information indicating a state of the estimation target from the feature information of the estimation target;

   wherein the estimating the physical quantity of the estimation target includes:

      generating the feature information of the estimation target from the proton position information and the atomic orbital information by using the transformation module;
      causing a quantum computer to execute calculation of the quantum circuit to acquire the state information from the quantum computer; and
      calculating the physical quantity of the estimation target based on the state information.

3. The estimation program according to claim 2,

   wherein the feature information of the estimation target includes a feature of each of a plurality of combinations of a proton and an electron included in the estimation target,

wherein the transformation module includes:

a first module (511) that calculates relative position information based on the atomic orbital information of the electron in each of the plurality of atoms included in the estimation target and a first parameter;
a second module (512, 513) that calculates a relative position of the electron with respect to the proton based on the proton position information and the relative position information for the proton and the electron included in each of the plurality of combinations; and
a third module (514 to 517) that generates the feature information of the estimation target based on the relative position of the electron with respect to the proton in each of the plurality of combinations; and

wherein a value of the first parameter is determined by training the machine learning model using the training data.

4. The estimation program according to claim 2,

wherein a position of the proton in each of the plurality of atoms included in the estimation target represents a position of an atomic nucleus of each of the plurality of atoms included in the estimation target,
wherein the feature information of the estimation target includes a feature of each of a plurality of combinations of an atomic nucleus and an electron included in the estimation target,
wherein the transformation module includes:

a first module (511) that calculates relative position information based on the atomic orbital information of the electron in each of the plurality of atoms included in the estimation target and a first parameter;
a second module (512, 513) that calculates a relative position of an electron with respect to the atomic nucleus based on the proton position information and the relative position information for the atomic nucleus and the electron included in each of the plurality of combinations; and
a third module (514 to 517, 1611) that generates the feature information of the estimation target based on the relative position of the electron with respect to the atomic nucleus of each of the plurality of combinations and a number of protons included in the atomic nucleus of each of the plurality of combinations; and

wherein a value of the first parameter is determined by training the machine learning model using the training data.

5. The estimation program according to any one of claims 2 to 4,

wherein the quantum circuit generates the state information based on the feature information of the estimation target and a second parameter, and
wherein a value of the second parameter is determined by training the machine learning model using the training data.

6. An estimation device comprising:

a receiving unit (111, 1212) that receives proton position information indicating a position of a proton in each of a plurality of atoms included in a first substance as an estimation target and atomic orbital information indicating an atomic orbital of an electron in each of the plurality of atoms;
an estimation unit (112, 1215) that estimates a physical quantity of the estimation target from the proton position information and the atomic orbital information by using a machine learning model (1223), the machine learning model being trained by using training data including information indicating a position of a proton in each of a plurality of atoms included in a second substance and information indicating an atomic orbital of an electron in each of the plurality of atoms included in the second substance; and
an output unit (113, 1211) that outputs an estimation result indicating the physical quantity of the estimation target.

7. The estimation device according to claim 6,

wherein the machine learning model includes:

a transformation module (411, 1511) that transforms the proton position information and the atomic orbital information into feature information of the estimation target; and
a quantum circuit (412) that generates state information indicating a state of the estimation target from the feature information of the estimation target; and

wherein the estimation unit generates the feature information of the estimation target from the proton position information and the atomic orbital information by using the transformation module, causes a quantum computer to execute calculation of the quantum circuit to acquire the state information from the quantum computer, and calculates the physical quantity of the estimation target based on the state information.

8. The estimation device according to claim 7,

   wherein the feature information of the estimation target includes a feature of each of a plurality of combinations of a proton and an electron included in the estimation target,
   wherein the transformation module includes:

      a first module (511) that calculates relative position information based on the atomic orbital information of the electron in each of the plurality of atoms included in the estimation target and a first parameter;
      a second module (512, 513) that calculates a relative position of the electron with respect to the proton based on the proton position information and the relative position information for the proton and the electron included in each of the plurality of combinations; and
      a third module (514 to 517) that generates the feature information of the estimation target based on the relative position of the electron with respect to the proton in each of the plurality of combinations; and

   wherein a value of the first parameter is determined by training the machine learning model using the training data.

9. The estimation device according to claim 7,

   wherein a position of the proton in each of the plurality of atoms included in the estimation target represents a position of an atomic nucleus of each of the plurality of atoms included in the estimation target,
   wherein the feature information of the estimation target includes a feature of each of a plurality of combinations of an atomic nucleus and an electron included in the estimation target,
   wherein the transformation module includes:

      a first module (511) that calculates relative position information based on the atomic orbital information of the electron in each of the plurality of atoms included in the estimation target and a first parameter;
      a second module (512, 513) that calculates a relative position of an electron with respect to the atomic nucleus based on the proton position information and the relative position information for the atomic nucleus and the electron included in each of the plurality of combinations; and
a third module (514 to 517, 1611) that generates the feature information of the estimation target based on the relative position of the electron with respect to the atomic nucleus of each of the plurality of combinations and a number of protons included in the atomic nucleus of each of the plurality of combinations; and

   wherein a value of the first parameter is determined by training the machine learning model using the training data.

10. The estimation device according to any one of claims 7 to 9,

   wherein the quantum circuit generates the state information based on the feature information of the estimation target and a second parameter, and
   wherein a value of the second parameter is determined by training the machine learning model using the training data.

11. An estimation method causing a computer to execute a process, the process comprising:

   receiving proton position information indicating a position of a proton in each of a plurality of atoms included in a first substance as an estimation target and atomic orbital information indicating an atomic orbital of an electron in each of the plurality of atoms;
   estimating a physical quantity of the estimation target from the proton position information and the atomic orbital information by using a machine learning model, the machine learning model being trained by using training data including information indicating a position of a proton in each of a plurality of atoms included in a second substance and information indicating an atomic orbital of an electron in each of the plurality of atoms included in the second substance; and
   outputting an estimation result indicating the physical quantity of the estimation target.

12. The estimation method according to claim 11,

   wherein the machine learning model includes:

      a transformation module (411, 1511) that transforms the proton position information and the atomic orbital information into feature information of the estimation target; and

a quantum circuit (412) that generates state information indicating a state of the estimation target from the feature information of the estimation target;

wherein the estimating the physical quantity of the estimation target includes:

generating the feature information of the estimation target from the proton position information and the atomic orbital information by using the transformation module; causing a quantum computer to execute calculation of the quantum circuit to acquire the state information from the quantum computer; and calculating the physical quantity of the estimation target based on the state information.

13. The estimation method according to claim 12,

wherein the feature information of the estimation target includes a feature of each of a plurality of combinations of a proton and an electron included in the estimation target, wherein the transformation module includes:

a first module (511) that calculates relative position information based on the atomic orbital information of the electron in each of the plurality of atoms included in the estimation target and a first parameter; a second module (512, 513) that calculates a relative position of the electron with respect to the proton based on the proton position information and the relative position information for the proton and the electron included in each of the plurality of combinations; and a third module (514 to 517) that generates the feature information of the estimation target based on the relative position of the electron with respect to the proton in each of the plurality of combinations; and

wherein a value of the first parameter is determined by training the machine learning model using the training data.

14. The estimation method according to claim 12,

wherein a position of the proton in each of the plurality of atoms included in the estimation target represents a position of an atomic nucleus of each of the plurality of atoms included in the estimation target, wherein the feature information of the estimation target includes a feature of each of a plurality of combinations of an atomic nucleus and an electron included in the estimation target, wherein the transformation module includes:

a first module (511) that calculates relative position information based on the atomic orbital information of the electron in each of the plurality of atoms included in the estimation target and a first parameter; a second module (512, 513) that calculates a relative position of an electron with respect to the atomic nucleus based on the proton position information and the relative position information for the atomic nucleus and the electron included in each of the plurality of combinations; and a third module (514 to 517, 1611) that generates the feature information of the estimation target based on the relative position of the electron with respect to the atomic nucleus of each of the plurality of combinations and a number of protons included in the atomic nucleus of each of the plurality of combinations; and

wherein a value of the first parameter is determined by training the machine learning model using the training data.

15. The estimation method according to any one of claims 12 to 14,

wherein the quantum circuit generates the state information based on the feature information of the estimation target and a second parameter, and wherein a value of the second parameter is determined by training the machine learning model using the training data.

FIG. 1

<u>101</u>

ESTIMATION DEVICE

111 — RECEPTION UNIT

112 — ESTIMATION UNIT

113 — OUTPUT UNIT

FIG. 2

```
                    ┌──────────┐
                    │  START   │
                    └────┬─────┘
                         │
                         ▼
201 ~  ┌─────────────────────────────────────────────┐
       │ RECEIVE PROTON POSITION INFORMATION AND     │
       │      ATOMIC ORBITAL INFORMATION             │
       └─────────────────────┬───────────────────────┘
                             │
                             ▼
202 ~  ┌─────────────────────────────────────────────┐
       │      ESTIMATE PHYSICAL QUANTITY OF          │
       │           ESTIMATION TARGET                 │
       └─────────────────────┬───────────────────────┘
                             │
                             ▼
203 ~  ┌─────────────────────────────────────────────┐
       │           OUTPUT ESTIMATION RESULT          │
       └─────────────────────┬───────────────────────┘
                             │
                             ▼
                    ┌──────────┐
                    │   END    │
                    └──────────┘
```

FIG. 3

304

301 ~ QUANTUM
COMPUTER

SERVER ~ 302

303 ~ TERMINAL DEVICE

FIG. 4

411 TRANS-FORMATION MODULE

412 QUANTUM CIRCUIT

PROTON ARRANGEMENT INFORMATION

ATOMIC ORBITAL INFORMATION

421 FEATURE TRANS-FORMATION CIRCUIT

422-1 QUANTUM TRANSFORMER

422-L QUANTUM TRANSFORMER

423 STATE TRANS-FORMATION CIRCUIT

STATE INFORMATION

FIG. 5

FIG. 6

FIG. 7

FIG. 8

FIG. 9

EP 4 726 724 A1

FIG. 10

713

HEA CIRCUIT

HEA (1, 1)
⌇
HEA (n, 1)

HEA (1, d)
⌇
HEA (n, d)

HEA (1)
⌇
HEA (q)

×h

EP 4 726 724 A1

FIG. 11

301

QUANTUM COMPUTER

1111 ⟩ COMMUNICATION INTERFACE

1112 ⟩ CONTROL DEVICE

1113 ⟩ QUANTUM SYSTEM

FIG. 12

302

SERVER

1211 ～ COMMUNICATION UNIT

1212 ～ ACQUISITION UNIT

1213 ～ QUANTIZATION UNIT

1214 ～ TRAINING UNIT

1215 ～ ESTIMATION UNIT

1216

STORAGE UNIT

1221 ～ ESTIMATION MODEL

1222 ～ TRAINING DATA SET

1223 ～ ESTIMATION MODEL

FIG. 13

```
                        ┌─────────────┐
                        │    START    │
                        └──────┬──────┘
                               │
                               ▼
1301 ┌───────────────────────────────────────────────────────┐
     │               INITIALIZE PARAMETER                     │
     └───────────────────────────┬───────────────────────────┘
                                 │
                                 ▼
1302 ┌───────────────────────────────────────────────────────┐
     │              GENERATE HAMILTONIAN                      │
     └───────────────────────────┬───────────────────────────┘
                                 │
                                 ▼
1303 ┌───────────────────────────────────────────────────────┐
     │           MAP GROUND STATE TO QUANTUM BIT              │
     └───────────────────────────┬───────────────────────────┘
                                 │
                                 ▼
1304 ┌───────────────────────────────────────────────────────┐
     │        GENERATE FEATURE INFORMATION OF SUBSTANCE       │
     └───────────────────────────┬───────────────────────────┘
                                 │
                                 ▼
1305 ┌───────────────────────────────────────────────────────┐
     │    TRANSMIT FEATURE INFORMATION OF SUBSTANCE           │
     │       AND INFORMATION OF QUANTUM CIRCUIT               │
     └───────────────────────────┬───────────────────────────┘
                                 │
                                 ▼
1306 ┌───────────────────────────────────────────────────────┐
     │              RECEIVE STATE INFORMATION                 │
     └───────────────────────────┬───────────────────────────┘
                                 │
                                 ▼
1307 ┌───────────────────────────────────────────────────────┐
     │        CALCULATE EXPECTED VALUE OF HAMILTONIAN         │
     └───────────────────────────┬───────────────────────────┘
                                 │
                                 ▼
1308 ┌───────────────────────────────────────────────────────┐
     │                 UPDATE PARAMETER                       │
     └───────────────────────────┬───────────────────────────┘
                                 │
                                 ▼
1309 ┌───────────────────────────────────────────────────────┐
     │              STORE ESTIMATION MODEL                    │
     └───────────────────────────┬───────────────────────────┘
                                 │
                                 ▼
                        ┌─────────────┐
                        │     END     │
                        └─────────────┘
```

FIG. 14

```
                    ┌─────────────┐
                    │    START    │
                    └──────┬──────┘
                           │
1401 ┌─────────────────────┴─────────────────────┐
     │ RECEIVE SUBSTANCE INFORMATION OF          │
     │ SUBSTANCE OF ESTIMATION TARGET            │
     └─────────────────────┬─────────────────────┘
                           │
1402 ┌─────────────────────┴─────────────────────┐
     │         GENERATE HAMILTONIAN              │
     └─────────────────────┬─────────────────────┘
                           │
1403 ┌─────────────────────┴─────────────────────┐
     │       MAP GROUND STATE TO QUANTUM BIT     │
     └─────────────────────┬─────────────────────┘
                           │
1404 ┌─────────────────────┴─────────────────────┐
     │    SELECT PROTON ARRANGEMENT INFORMATION  │
     └─────────────────────┬─────────────────────┘
                           │
1405 ┌─────────────────────┴─────────────────────┐
     │   GENERATE FEATURE INFORMATION OF SUBSTANCE│
     └─────────────────────┬─────────────────────┘
                           │
1406 ┌─────────────────────┴─────────────────────┐
     │ TRANSMIT FEATURE INFORMATION OF           │
     │ SUBSTANCE AND INFORMATION OF QUANTUM CIRCUIT│
     └─────────────────────┬─────────────────────┘
                           │
1407 ┌─────────────────────┴─────────────────────┐
     │         RECEIVE STATE INFORMATION         │
     └─────────────────────┬─────────────────────┘
                           │
1408 ┌─────────────────────┴─────────────────────┐
     │   CALCULATE EXPECTED VALUE OF HAMILTONIAN │
     └─────────────────────┬─────────────────────┘
                           │
1409              ◇ HAS ALL PROTON ARRANGEMENT ◇ ── NO
                    INFORMATION BEEN SELECTED?
                           │ YES
                           │
1410 ┌─────────────────────┴─────────────────────┐
     │         GENERATE ESTIMATION RESULT        │
     └─────────────────────┬─────────────────────┘
                           │
1411 ┌─────────────────────┴─────────────────────┐
     │         TRANSMIT ESTIMATION RESULT        │
     └─────────────────────┬─────────────────────┘
                           │
                    ┌──────┴──────┐
                    │     END     │
                    └─────────────┘
```

FIG. 15

FIG. 16

FIG. 17

Europäisches
Patentamt
European
Patent Office
Office européen
des brevets

# EUROPEAN SEARCH REPORT

**Application Number**

EP 25 20 4061

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2024/095572 A1 (NISHIDA YASUTAKA [JP] ET AL) 21 March 2024 (2024-03-21) * the whole document * ----- | 1-15 | INV. G16C20/30 G06N10/20 G06N10/60 |
| A | US 2023/359929 A1 (SHMILOVICH KIRILL [US] ET AL) 9 November 2023 (2023-11-09) * the whole document * ----- | 1-15 | ADD. G16C10/00 G16C20/70 |
| A | US 2023/409895 A1 (LIU HONGBIN [US] ET AL) 21 December 2023 (2023-12-21) * the whole document * ----- | 1-15 | G16C20/90 |
| A | WO 2020/095051 A2 (GTN LTD [GB]) 14 May 2020 (2020-05-14) * the whole document * ----- | 1-15 | |
| A | CEREZO M. ET AL: "Variational quantum algorithms", NATURE REVIEWS PHYSICS, vol. 3, 12 August 2021 (2021-08-12), pages 625-644, XP093371135, DOI: https://doi.org/10.1038/s42254-021-00348-9 * the whole document * ----- | 1-15 | **TECHNICAL FIELDS SEARCHED (IPC)** G16C G06E G06N |

The present search report has been drawn up for all claims

| Place of search | Date of completion of the search | Examiner |
|---|---|---|
| The Hague | 28 February 2026 | Godzina, Przemyslaw |

EPO FORM 1503 03.82 (P04C01)

## ANNEX TO THE EUROPEAN SEARCH REPORT
## ON EUROPEAN PATENT APPLICATION NO.

EP 25 20 4061

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2026

| Patent document cited in search report | | Publication date | Patent family member(s) | | Publication date |
|---|---|---|---|---|---|
| US 2024095572 | A1 | 21-03-2024 | JP | 2024044001 A | 02-04-2024 |
| | | | US | 2024095572 A1 | 21-03-2024 |
| US 2023359929 | A1 | 09-11-2023 | NONE | | |
| US 2023409895 | A1 | 21-12-2023 | US | 2023409895 A1 | 21-12-2023 |
| | | | WO | 2023244455 A1 | 21-12-2023 |
| WO 2020095051 | A2 | 14-05-2020 | US | 2021398621 A1 | 23-12-2021 |
| | | | WO | 2020095051 A2 | 14-05-2020 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2024500182 A

**Non-patent literature cited in the description**

- **I. VON GLEHN et al.** A Self-Attention Ansatz for Ab-initio Quantum Chemistry. *arXiv:2211. 13672v2*, 2023
- **A. CERVERA-LIERTA et al.** Meta-Variational Quantum Eigensolver: Learning Energy Profiles of Parameterized Hamiltonians for Quantum Simulation. *PRX QUANTUM 2, 020329*, 2021
- **J. SHI et al.** QSAN: A Near-term Achievable Quantum Self-Attention Network. *arXiv:2207. 07563v4*, 2023